# EUROPEAN PATENT APPLICATION

(11) **EP 0 791 596 A1**
(43) Date of publication of application: **27.08.1997**
(21) Application number: 96301161.4
(22) Date of filing: 21.02.1996
(51) Int. Cl.: C07D 501/06, C07D 277/20, C07D 257/04, A61K 31/545

(54) **Method for manufacture of cephalosporins and intermediates thereof**

(71) Applicant: LUPIN LABORATORIES LIMITED, Bombay, Maharashtra - 400098 (IN)
(72) Inventor: Data, Debashish, Dr., Lupin Lab. Ltd., Mandideep, Dist Raisen, M.P. 462 046 (IN); George, Vinod, Lupin Lab. Ltd., Mandideep, Dist Raisen, M.P. 462 046 (IN); Rai, Bishwa P., Lupin Lab. Ltd., Mandideep, Dist Raisen, M.P. 462 046 (IN)
(74) Representative: Thornley, Rachel Mary

(57) **Abstract**

This invention relates to reactive derivatives of 2-(2-aminothiazol-4-yl)-2-methoxyimino acetic acid and 1H-tetrazol-1-acetic acid of the following general formula I, 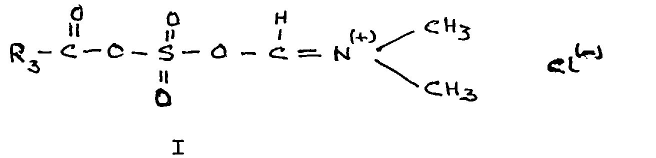
- wherein: R₃ = 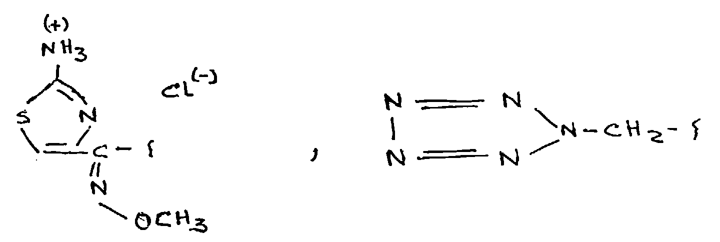
as well as to use thereof in the manufacture of cephalosporin antibiotics such as cefotaxime, ceftriaxone and cefazolin.

## Description

This invention relates to reactive derivatives of 2-(2-aminothiazol-4-yl)-2-methoxyimino acetic acid and 1H-tetrazol-1-acetic acid of the following general formula I, 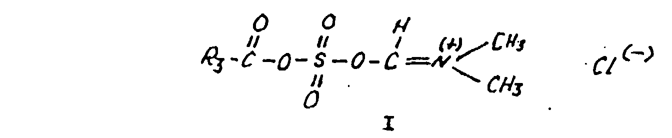
- wherein: R₃ =

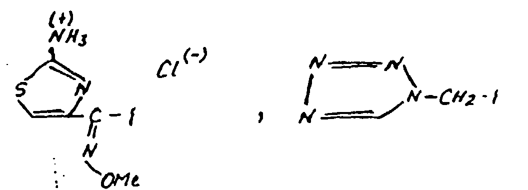

The present invention also relates to a process for the production thereof as well as to a process for the use thereof in the manufacture of cephalosporin antibiotics such as cefotaxime, ceftriaxone and cefazolin.

It is known in the art to synthesize different cephalosporin antibiotics for instance compounds of formula II which generally comprise of amidification of 7-position of appropriate cephalosporin nucleus with or without substitution at 3α-position, with the desired addendums. 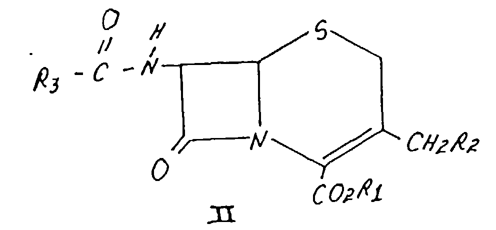
- wherein: R₁ = H, carboxylic protecting group;
R₂ = acetoxy, 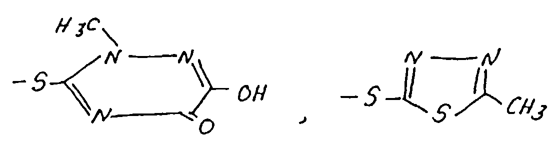 R₃ =

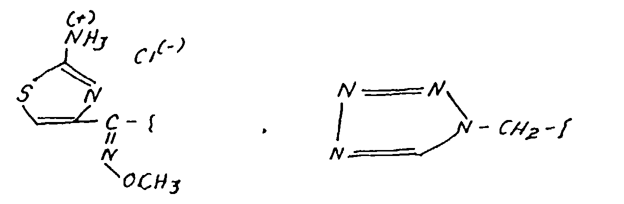

Usually, the synthesis by such known processes of various cephalosporin antibiotics comprises of acylation of 7-aminocephalosporanic acid(7-ACA) or 7-amino desacetoxy-cephalosporanic acid (7-ADCA) derivatives with a reactive derivative such as an acid chloride, an acid anhydride, an activated ester etc., of the addendum acid for instance of formula III and IV.

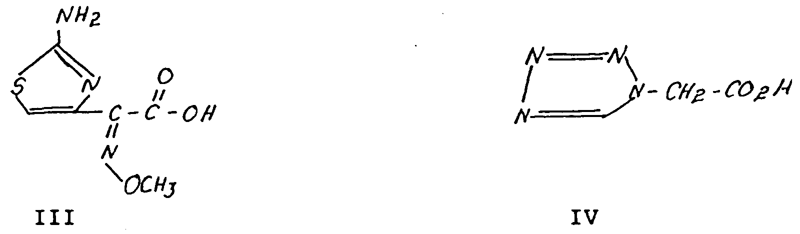

The above can be described schematically as follows:

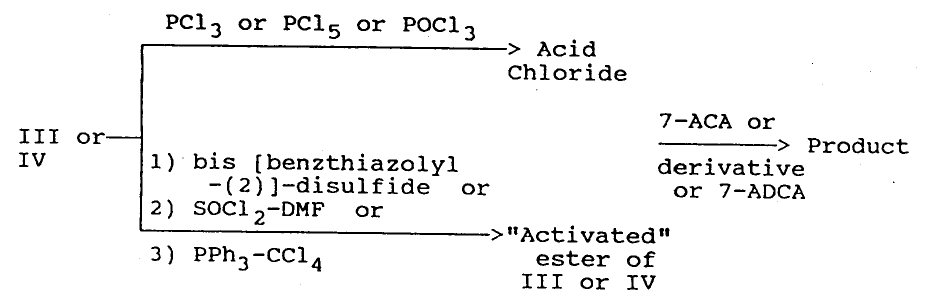

A number of methods utilising this concept has been reported for the functionalisation of position 7 of the desired cephalosporin nucleus and these are summarised below.

US 4 152 432 describes acylation of 7-ACA with an acid chloride of formula V in which the amino group in the thiazole ring is protected. The amino group is subsequently deprotected generally by hydrolysis or hydrogenolysis.

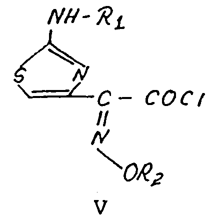

Because of the inherent inefficiency of amidification of the 7-amino group of cephalosporin nucleus and also of the steps involved in protection and deprotection of amino group of the thiazolyl ring, the overall yields are low and far from satisfactory.

JP 52-102096, JP 53-157596 and GB 2 025 933 utilise the same chemistry i.e. formation of 2-(2-aminothiazol-4-yl)-2-oxyimino acetyl chloride either with PCl₃, PCl₅, SOCl₂ or POCl₃.

Needless to say that the protection and deprotection of the amino group of the thiazolyl ring are involved in such conventional synthesis techniques for cephalosporin antibiotics.

Besides, protection and deprotection of the amino function, the resulting acid chloride eg. 2-(2-aminothiazol-4-yl)-2-methoxyimino acetyl chloride, 1H-tetrazol-1-acetyl chloride, are unstable creating further complication for the synthesis of the desired cephalosporin antibiotics.

US 3 954 745 utilises the same concept i.e formation of 1H-tetrazol-1-acetyl chloride in dimethylacetamide and coupling the said acid chloride with the DMF-solvate of the hydrochloride of 7-aminocephalosporin of the following formula VI as shown below to yield cefazolin.

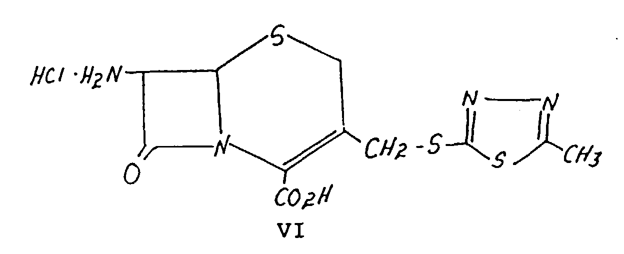

However, yields are low. Also, the acylating agent, 1H-tetrazol-1-acetyl chloride because of its inherent instability is difficult to isolate and store at room temperature. Another limitation of this process is that acylation is to be carried out in strictly anhydrous condition.

US 5 317 099 describes a process for the synthesis of β-lactam derivatives such as cefotaxime, ceftriaxone in which silylated 7-ACA is acylated with acyloxyphosphonium chloride derivative of 2-(2-aminothiazol-4-yl)-2-syn-methoxyimino acetic acid which in turn is prepared from triphenyl phosphine(TPP), hexachloroethane or carbontetrachloride and 2-(2-aminothiazol-4-yl)-2-syn-methoxyimino acetic acid.

Since, triphenyl phosphine(TPP) is used as a reactant, the overall cost of coupling the addendum III to the cephalosporin nucleus becomes high.

EP 037 380 describes a process for the synthesis of cephalosporin antibiotics eg. cefotaxime and ceftriaxone which comprises acylation of 7-ACA derivatives with an intermediate of formula VII,

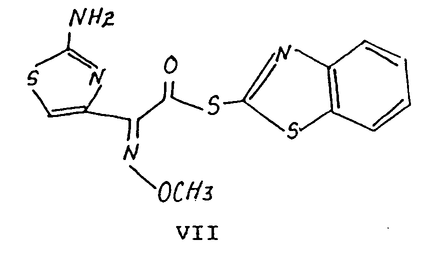

However, the synthesis of the 2-(2-aminothiazol-4-yl)-2-methoxyimino acetic acid benzthiazolyl thioester from 2-(2-aminothiazol-4-yl)-2-methoxyimino acetic acid and bis [benzthiazolyl-(2)]disulfide, involves the use of costly condensation aid such as triphenyl phosphine(TPP), rendering the whole synthesis very costly.

US 5 037 988 describes a process for the production of cephalosporins in particular cefotaxime and ceftriaxone, in which an activated form of an organic acid i.e 2-(2-aminothiazol-4-yl)-2-oximinoacetylsulfite dialkylformiminium halide hydrohalide of following formula VIII; 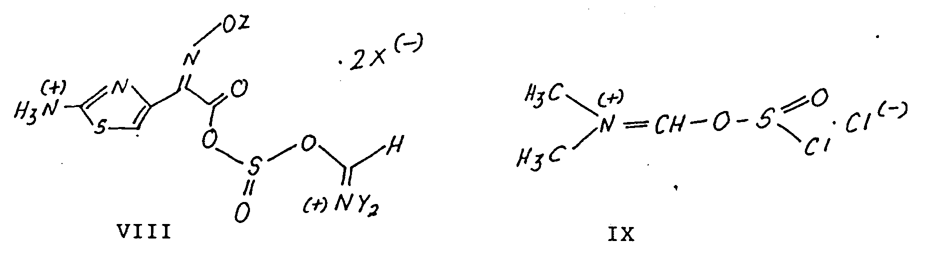 is coupled with a 7-aminocephalosporanic acid derivative. The compound of formula VIII was prepared by reacting 2-(2-aminothiazol-4-yl)-2-oximino acetic acid with dimethyl formiminium chloride chlorosulfite of formula IX which in turn was prepared by reacting approximately equimolar quantities of thionyl chloride and dimethylformamide at room temperature in specific solvents only like benzene or toluene, and hence a limitation.

However, poor results have been obtained when an attempt was made to prepare the compound of formula IX in solvents such as chloroform and dichloromethane. In these solvents the compound IX did not separate out in its characteristic form of insoluble oil, as the polarity of these solvents does not allow the formation of these reactants.

Thus, it is evident that the procedures described in the prior art for the preparation of 7-acylamino cephalosporanic acid derivatives are either complex involving protection or deprotection or costly or have other limitations.

The present invention provides reactive derivatives of general formula I which would be suitable for use in the manufacture of cephalosporin antibiotics.

Another aspect of the present invention is directed to provide a process for the synthesis of the reactive derivatives of 2-(2-aminothiazol-4-yl)-2-methoxyimino acetic acid(III) and 1H-tetrazol-1-acetic acid(IV) of general formula I which would be more simply carried out than known methods.

Yet further aspect of the present invention is to provide a process for the synthesis of cephalosporin antibiotics such as cefotaxime, ceftriaxone and cefazolin by which the protection and deprotection of the amino group of the thiazolyl ring reported in the known art may be avoided.

An object of the present invention is to provide a process for the synthesis of cephalosporin antibiotics of the type mentioned above which may be more simply carried out and more cost effective than known methods.

Yet another further object of the present invention is to provide for the synthesis of cephalosporin antibiotics of the type mentioned above which would favour better and consistent yield of cephalosporin antibiotics with desired characteristics for its use as an antibiotic.

Thus, according to one aspect of the present invention compounds of formula I are produced by a process comprising reacting dimethyl formiminium chloride chlrosulphate (DFCCS) of formula XI with 2-(2-aminothiazol-4-yl)-2-methoxyimino acetic acid(III) or 1H-tetrazol-1-acetic acid(IV) in a solvent such as dichloromethane at a temperature ranging from -30°C to 15°C to yield the compound of formula I.

In the instance in formula I 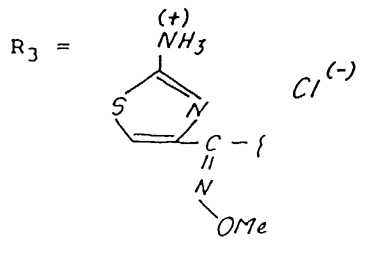 the above reactive derivative of formula I is a reactive derivative of 2-(2-aminothiazol-4-yl)-2-methoxyimino acetic acid of formula Ia,

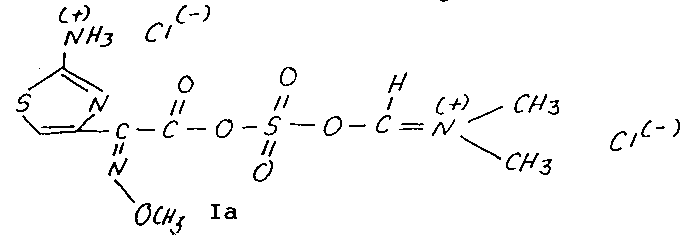

Further, in the instance in formula I, 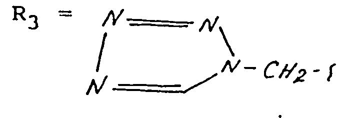 the above reactive derivative of formula I is a reactive derivative of 1H-tetrazol-1-acetic acid of formula Ib,

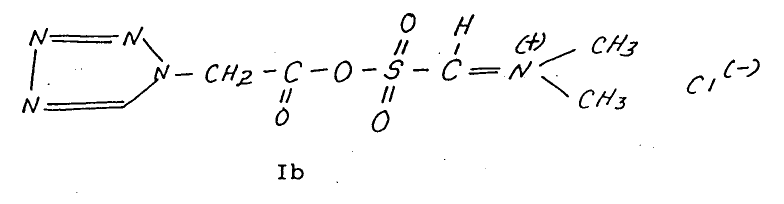

In accordance with a further aspect of the invention there is provided a process for the preparation of the desired cephalosporin antibiotics which comprises reacting silylated 7-aminocephalosporanic acid derivatives of formula X, 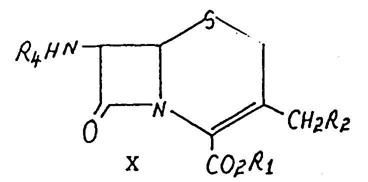
- in which: R₁ = carboxylic protecting group or hydrogen
R₂ = acetoxy,
R₄ = silyl group or hydrogen
with a reactive derivative of 2-(2-aminothiazol-4-yl)-2-methoxyimino acetic acid of formula Ia or 1H-tetrazol-1-acetic acid of formula Ib, in dichloromethane at a temperature ranging from -70°C to -50°C.

In accordance with the invention, compounds of formula Ia & Ib are prepared by reacting N, N-dimethyl formiminium chloride chlorosulphate (DFCCS) of formula XI, with 2-(2-aminothiazol-4-yl)-2-methoxyimino acetic acid (III) or 1H-tetrazol-1-acetic acid (IV).

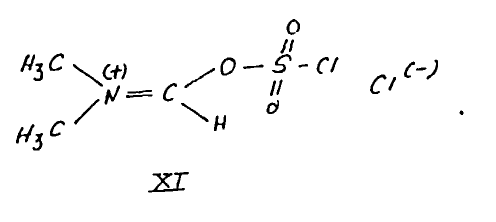

DFCCS(XI) is a known compound and described in the literature viz Z. Chem, 6(4), 148 (1966), J.C.S. Perkin Trans I, 2004-2007 (1972); Bull Chem Soc Jpn, 58, 1063-1064; Adv. -Org. Chem. 9(2), 5, (1979); Synthetic Reagents Vol.4, 388 - 389; Angew Chem Internal Edit, 1(12), 647 (1962).

While DFCCS(XI) prepared by any known process might be used but the inventors have found that best results are obtained when DFCCS(XI) is prepared by the following process.

The preferred process of obtaining the DFCCS(XI) comprises adding sulfuryl chloride to dimethylformamide at -20°C. The temperature is raised to 0°C at which the solid adduct crystallised out, which is vigorously stirred for one hour followed by addition of dichloromethane to the resulting reaction mixture. The temperature was raised to 15°C - 20°C and at this temperature the solid crystals melt resulting in the formation of immiscible layer of the desired adduct i.e XI.

Such mode of preparation of DFCCS(XI) is illustrated in the following scheme A:

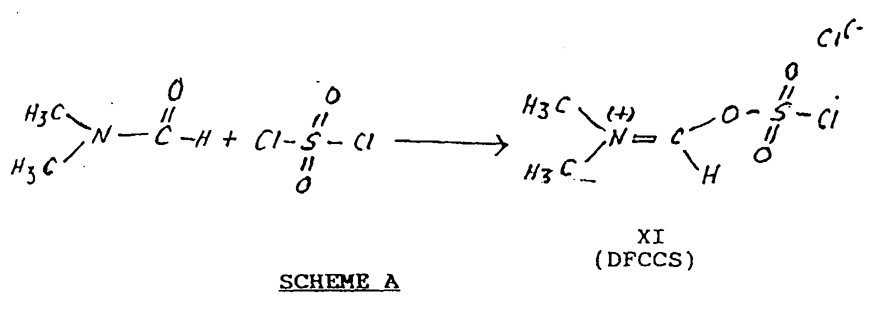

The DFCCS(XI) adduct thus obtained by the preferred process of the invention is found to be advantageous in use in the process of manufacture of the reactive derivatives of formula X in accordance with the objective of the invention for the reasons given below.
i) Unlike the complex IX used in the prior art, DFCCS (XI) used in the process of the present invention remains stable and does not get converted to the normal Vilsmeier's reagent. It has been observed that DFCCS(XI) of the present invention is apparently more stable than DFCS(IX) described in US patent 5 037 988. In particular it is found that the thus obtained DFCCS(XI) used in the process of the invention is distinct from thionyl chloride-DMF adduct i.e. dimethylformiminium chloride chlorosulfite(IX) known in the art. The melting point of the latter is 138° - 140° C [Helv. Chim Acta, 62, 1655 (1959)] while that of the DFCCS adduct(XI) is 40°C - 41° C [Z. Chem 6(4), 148(1966)]
ii) The DFCCS (XI) used in the process of the invention can be prepared in any solvent such as benzene, toluene, acetonitrile or dichloromethane, and preferably in the absence of solvents. This is advantageous and clearly distinct from the thionyl chloride - DMF adduct i.e dimethylformiminium chloride chlorosulfite described in US patent 5 037 988 which cannot be prepared in solvents such as chloroform or dichloromethane, since these solvents facilitate complete or partial conversion of dimethyl formiminium chloride chlorosulfite to normal Vilsmeier's reagent.
iii) It has been found that sulfuryl/DMF adduct DFCCS of formula XI is more stable than DFCS adduct of formula IX made from Thionyl chloride and DMF. Thus, when DFCCS (XI) adduct was kept at ambient temperature for 16 hrs. and used for further complexation with compounds of formula III and IV for synthesis of the activated ester required for the final acylation reaction, the drop in yield in the final antibiotic was about 26% (85% when used fresh and 59% after storage of DFCCS for 16 hrs.). Similarly when DFCS adduct was kept at ambient temperature for 16 hrs. and further processed for synthesis of the final antibiotic, the drop in yield was about 35% (80% when used fresh and 45% when used after 16 hrs.). Hence DFCCS adduct of formula XI obtained by the preferred process described above is having superior stability compared to DFCS adduct of formula IX and this is a great advantage for cost effective manufacture of cephalosporin antibiotic like Cefotaxime, Ceftriaxone and Cefazolin.
   Thus, the use of DFCCS(XI) for synthesis of compound of formula Ia or Ib, the activated ester of compounds of formula III and IV provides a more practical, cost effective and safe method for manufacture of the desired cephalosporin antibiotics like cefotaxime, ceftriaxone and cefazolin.
   The process of manufacture of the desired cephalosporin antibiotic according to the improved process of the invention basically involves the following :
   In Step I the 2-(2-aminothiazol-4-yl)-2-syn-methoxyimino acetic acid(III) was activated with N,N-dimethylformiminium chloride chlorosulphate(XI) in dichloromethane at a temperature ranging from -30°C to -15°C to yield reactive form Ia.
   In step II the reactive form Ia was treated with silylated 7-aminocephalosporanic acid (X) at a temperature ranging from -70°C to -30°C preferably -55°C to yield the desired cephalosporins such as cefotaxime, ceftriaxone.

   The use of conventional silylating agents such as hexamethyldisilazane, trimethylchlorosilane, bis (trimethyl) silylacetamide is considered to be appropriate for silylation of 7-ACA. Alternatively 7-amino-cephalosporanic acid can also be used in the form of its quaternary salt with tetramethyl guanidine.
   The basic compounds used as acid scavanging agent to capture HCl released during silylation include N,N-dimethylaniline, diethylamine, pyridine preferably N,N-dimethylaniline.
   The process of the present invention described above may be represented in scheme B : 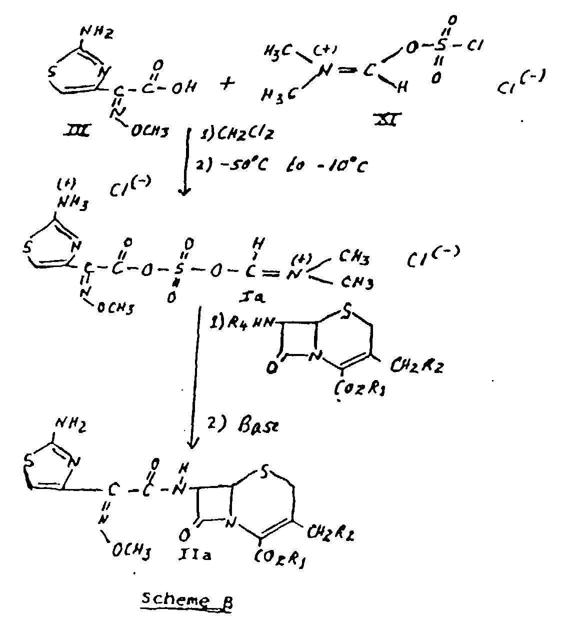
   Cefazolin is prepared from 7-amino-3-(2-methyl-1,3,4-thiadiazol-5-yl)thiomethyl-3-cephem-4-carboxylic acid and reactive derivative of 1H-tetrazol-1-acetic acid of formula Ib in a manner similar to that described for the preparation of cefotaxime, ceftriaxone.
   The preparation of cefazolin is illustrated in the following scheme C: 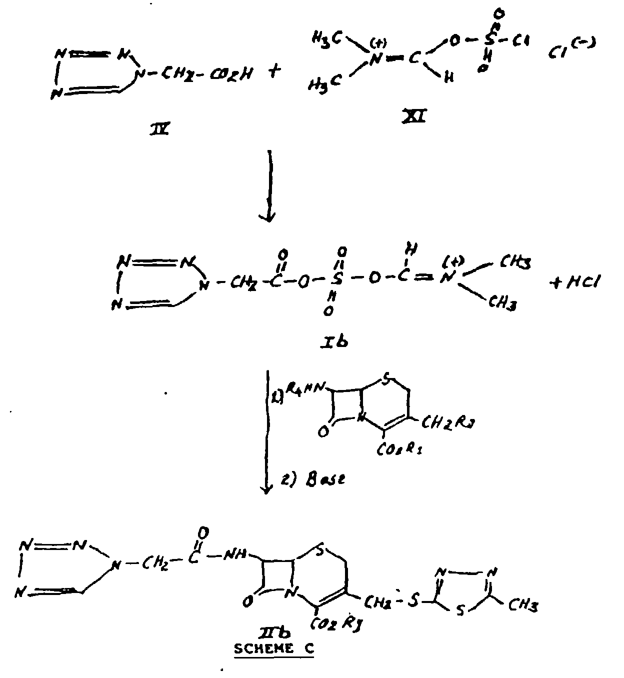
   The process of the invention, its objects and advantages will be further apparent from the non limiting examples illustrated hereunder:

### EXAMPLES

### Example 1

### Preparation of 7-[(2-(2-aminothiazol-4-yl)-2-syn-methoxyimino)acetamido] cephalosporanic acid (cefotaxime).

A) Activation of the 2-(2-aminothiazol-4-yl)-2-syn-methoxyimino acetic acid.
   11.9 g of sulfuryl chloride was added dropwise to 6.4 g of dimethylformamide at -20°C. The temperature was slowly raised to 0°C at which the solid adduct crystal lised out. This was stirred vigorously for 1 hour and 50 ml of dichloromethane was added to the solid crystals. The temperature was raised to 15° - 20°C and at this temperature the crystallised adduct melted and formed an immiscible layer with dichloromethane. The lower portion (N,N-Dimethyl forminium chloride chloro sulphate) was added to a pre cooled slurry of 16.02 g of 2-(2-aminothiazol-4-yl)-2-syn-methoxyimino acetic acid in 150 ml of dichloromethane at -20°C to get a clear solution, which was kept for 1 hour at this temperature.
B) Preparation of 7-amino cephalosporanic acid solution.
   20.0g of 7-aminocephalosporanic acid was taken in 150 ml of dichloromethane and 12.24g of hexamethyl-disilazane was added to it, followed by refluxing for two hours. The clear solution obtained was cooled to 10°C and 12.1 ml of dimethylaniline was added to it, followed by cooling to -55°C.
C) Acylation
   The reaction mixture (A) was cooled to -55°C and the reaction mixture (B) was added to it to get a clear solution. The temperature was maintained at -55°C for 10 minutes. The quantification of the condensed mass showed the formation of 82.5% of cefotaxime acid.
   PMR (DMSO-d₆) δ ppm : 2.00(s), 3.3(q), 3.8(s), 4.9(q), 4.97(q), 5.60(2d), 6.70(s), 7.2(bs), 9.47(d).
D) Isolation of 7-[(2-(2-aminothiazol-4-yl)-2-syn-methoxyimino)acetamido] cephalosporanic acid.
   200 ml of water was added to the above condensed mass and the temperature was brought to 25°C in 20 minutes. At this temperature, the pH of the hydrolysed mass was brought to 6.5 by using triethylamine. The aqueous layer was taken and 40 ml of 85% formic acid was added at 30°C. The product in the formic acid solvate form started precipitating after 10 minutes. This was cooled to 5°C and was stirred for one hour and then filtered to yield 29 g (87%) of the formic acid solvate with a purity of 92%.
   UV max = 233.0nm (in water)
E) Conversion of formic acid solvate to isopropyl alcohol solvate of cefotaxime:
   The above said formic acid solvate was taken in 175 ml of isopropyl alcohol and heated to 50°C for 4-5 hours. The isopropyl alcohol solvate thus formed was cooled to 5°C and then filtered to yield 26.97g (81%) of the isopropyl alcohol solvate with a purity of 92%. MP = 202-205°C.
   a) IR : (main bands) in cm ⁻¹.
      3420 (-NH₂), 3340 (-NH, -NH₂), 1760 (-C=O lactam),1730 (-C=O, carboxylic, 1650 (-C(=O)-NH), 1385 - 1355 (-O-CO-CH₃).
   b) UV Characteristics :
      UV max = 235.5nm (in water), E(1%,1 cm) = 376.00
   c)Specific rotation :
      [α]²⁰_{D} = +61.232° (C =1% aqueous solution)
F) Conversion of Isopropyl alcohol solvate of cefotaxime to cefotaxime sodium:
   10.0g of the above solvate was taken in 60 ml of methanol and 11 ml of water and the mixture was cooled to +5°C. To this was slowly added 1.8 g of sodium acetate dissolved in 20 ml methanol to get a clear solution. To the above solution 400 ml of isopropyl alcohol was added within one hour to get cefotaxime sodium in the crystal form.

### Example 2

### Preparation of 7-[(2-(2-aminothiazol-4-yl)-2-syn-methoxyimino) acetamido] cephalosporanic acid (cefotaxime).

Same procedure as in example 1 was followed, but 400 ml of dichloromethane was used for the activation of 2-(2-amino thiazol-4-yl)-2-syn-methoxyimino acetic acid. Cefotaxime was prepared in 81.4% yield.

### Example 3

### Preparation of 7-[(2-(2-aminothiazol-4-yl)-2-syn-methoxyimino) acetamido] cephalosporanic acid (cefotaxime).

A) Activation of 2-(2-aminothiazol-4-yl)-2-syn-methoxyimino acetic acid:
   Same procedure as in example 1 was followed.
B) Preparation of 7-aminocephalosporanic acid solution:
   Same procedure as in example 1 was followed.
C) Acylation:
   The reaction mixture (A) was cooled to -70°C and reaction mixture (B) was added to it to get a clear solution. The temperature of the condensed mass was maintained at -70°C for 10-15 minutes. The quantification of the condensed mass showed the formation of 75.5% of cefotaxime.
D) Isolation of 7-[(2-(2-aminothiazol-4-yl)-2-syn-methoxyimino) acetamido]cephalosporanic acid.
   Same procedure as in example 1 was followed.

### Example 4

### Preparation of 7-[(2-(2-aminothiazol-4-yl)-2-syn-methoxyimino)acetamido] cephalosporanic acid (cefotaxime)

A) Activation of 2-(2-aminothiazol-4-yl)-2-syn-methoxyimino acetic acid.
   19.8 g of sulfuryl chloride was added dropwise to 10.7g of dimethylformamide at -20°C. The temperature was slowly raised to 0°C at which white solid crystallises out. The reaction mixture was stirred vigorously for one hour. 60 ml of dichloromethane was added to the solid crystals and the temperature was brought to 15°-20°C. At this temperature, the solid crystals melted to form immiscible layer. The lower layer was added to a pre-cooled slurry of 15.08 g of 2-(2-aminothiazol-4-yl)-2-syn-methoxyimino acetic acid in 120 ml of dichloromethane at -20°C to get a clear solution which was kept for one hour at this temperature.
B) Preparation of 7-aminocephalosporanic acid solution:
   Same procedure as in example 1 was followed.
C) Acylation:
   The reaction mixture A was cooled to -55°C and the reaction mixture B was added to it to get a clear solution. The temperature was maintained at -50°C for 10 minutes. The quantification of the condensed mass showed only 36.3% of cefotaxime.
D) Isolation of 7-[(2-(2-aminothiazol-4-yl)-2-syn-methoxyimino)acetamido] cephalosporanic acid:
   Same procedure as in example 1 was followed.

### Example 5 :

### Preparation of 7-[(2-(2-aminothiazol-4-yl)-2-syn-methoxyimino)acetamido] cephalosporanic acid (cefotaxime)

A) Activation of 2-(2-aminothiazol-4-yl)-2-syn-methoxyimino acetic acid:
   10.9 g of sulfuryl chloride was added dropwise to 5.9 g of dimethylformamide at -20°C. The temperature was slowly increased to 0°C at which the solid adduct crystallised out. This was vigorously stirred for 1 hour at this temperature. To the solid crystals, 30 ml of dichloromethane was added and the temperature was raised to 15°-20°C. At this temperature the solid crystals melted and formed an immiscible layer with dichloromethane. The lower layer was added to pre-cooled slurry of 15.08 g of 2-(2-aminothiazol-4-yl)-2-syn-methoxyimino acetic acid in 120 ml of dichloro methane at -20°C to get a clear solution which was kept for one hour at the same temperature.
B) Preparation of 7-amino cephalosporanic acid solution :
   Same procedure as in example 1 was followed.
C) Acylation :
   The reaction mixture A was cooled to -55°C and reaction mixture B was added to it to get a clear solution. The temperature of the condensed mass was kept at -50°C for 10 minutes. The quantification of the condensed mass showed 60% of cefotaxime.
D) Isolation of 7-[(2-(2-aminothiazol-4-yl)-2-syn-methoxyimino)acetamido]cephalosporanic acid:
   Same procedure as in example 1 was followed.

### Example 6 :

### Preparation of 7-[(2-(2-aminothiazol-4-yl)-2-syn-methoxyimino)acetamido]cephalosporanic acid (cefotaxime).

A) Activation of 2-(2-aminothiazol-4-yl)-2-syn-methoxyimino acetic acid.
   Same procedure as in example 1 was followed.
B) Preparation of 7-aminocephalosporanic acid solution:
   Same procedure as in example 1 was followed.
C) Acylation:
   The reaction mixture A was cooled to -55°C and the reaction mixture B was added to it to get a clear solution. The temperature was maintained at -50°C for 30 minutes, instead of 10 minutes. The quantification of condensed mass showed 55% of cefotaxime.
D) Isolation of 7-[(2-(2-aminothiazol-4-yl)-2-syn-methoxyimino)acetamido] cephalosporanic acid :
   Same procedure as in example 1 was followed.

### Example 7 :

### Preparation of 7-[(2-(2-aminothiazol-4-yl)-2-syn-methoxyimino)acetamido] cephalosporanic acid (Cefotaxime).

A) Activation of 2-(2-aminothiazol-4-yl)-2-syn-methoxyimino acetic acid:
   Same procedure as in example 1 was followed.
B) Preparation of 7-aminocephalosporanic acid solution:
   20.0 g of 7-aminocephalosporanic acid was taken in 150 ml of dichloromethane and 12.24 g of hexamethyldisilazane was added to it followed by refluxing for 2 hours. The clear solution obtained was cooled to 10°C and 9.4 g of acetamide was added to it followed by cooling to -55°C.
C) Acylation :
   Same procedure as in example 1 was followed. Quantification of the condensed mass showed 60% of cefotaxime.
D) Isolation of 7-[(2-(2-aminothiazol-4-yl)-2-syn-methoxyimino)acetamido] cephalosporanic acid:
   Same procedure as in example 1 was followed.

### Example 8 :

### Preparation of 7-((2-(2-aminothiazol-4-yl)-2-syn-methoxyimino acetamido) cephalosporanic acid (cefotaxime).

A) Activation of the 2-(2-aminothiazol-4-yl)-2-syn-methoxyimino acetic acid.
   11.9 g of sulfuryl chloride was added dropwise to 6.4 g of dimethylformamide at -20°c. The temperature was slowly raised to 0°c at which the solid adduct crystallises out. This was stirred vigorously for 1 hour and 50 ml of dichloromethane was added the solid crystals. The temperature was raised to 15-20°c and at this temperature the crystallised adduct melts and forms and immiscrible layer with dichloromethane. The lower portion (N,N-Dimethyl forminium chloride chloro sulphate) was added to a pre cooled slurry of 16.25 g of 2-(2-aminothiazol-4-ul)-2-syn-methoxyimino acetic acid in 150 ml of dichloromethane at -20°c to get a clear solution, which was kept for 1 hour at this temperature.
B) Preparation of 7-amino cephalosporanic acid solution.
   20.0 g of 7-amino cephalosporanic acid was taken in 120 ml of CH₂Cl₂ and 10.65 gm Hexamethyl disilazane and 0.1 gm imidazole was added to it. Start refluxing for 4.0 hours under N₂ atmosphere. (After 30 minutes to refluxing, clear solution obtained.) Cooled to 10°c and 12.1 ml Dimethyl aniline was added followed by colling to 55°c.
C) Acylation :
   The reaction mixture (A) was cooled to -55°c and the reaction mixture (B) was added to it to get a clear solution. The temperature was maintained at -55°c for 10 minutes. The quantitification of the condensed mass showed the formation of 90-95% of cefotaxime acid depending on the potency of the starting product (7-ACA).
D) Isolation of 7-((2-aminothiazol-4-yl)-2-syn-methoxyimino)acetamido) cephalosporanic acid.
   80 ml of water was added to the above condensed mass and the temperature was brought to 25°c in 20 minutes. At this temperature, the pH of the hydrolysed mass was brought to 6.5 by using triethylamine. The aqueous layer separated. Organic layer was extracted with 20x2 ml DM Water. Combined aqueous layer & extraction was taken and 40 ml of 85% formic acid was added at 30°c. The product in the formic acid solvate form starts precipitating after 10 minutes. This was cooled to 0-5°c and was stirred for one hour and then filtered to yield 30 gm (87%) of the formic acid solvate with a purity of 92%.
E) Conversion of formic acid slovate to isopropyl alcohol solvate of cefotaxime :
   Same procedure as in example 1 was followed.
F) Conversion of Isopropyl alcohol solvate of cefotaxime to cefotaxime sodium.
   Same procedure as in example 1 was followed.

### Example 9 :

### Preparation of 7-(1-(1H)-tetrazolylacetamido)-3-[2- (5-methyl-1,3,4-thiadiazolyl)thiomethyl]-3-cephem-4-carboxylic acid (Cefazolin).

A) Activation of 1H-tetrazol-1-acetic acid: 9.41 g of sulfuryl chloride was added dropwise to 5.09 g of dimethylformamide at -20°C. The temperature was slowly raised to 0°C at which the solid adduct crystallised out. To this 50 ml of dichloromethane was added and the solid crystals melted to form an immiscible layer. The lower layer was added to 8.9 g of 1H-tetrazol-1-acetic acid in 120 ml of dichloromethane at -20°C. The temperature was raised to 0°C which resulted in the formation of a turbid reaction mass. This reaction mass was kept at 0°C for one hour.
B) Preparation of 7-amino-3-[2-(5-methyl-1,3,4-thiadiazolyl)thiomethyl]-3-cephem-4-carboxylic acid solution:
   20.0 g of 7-amino-3-[2-(5-methyl-1,3,4-thiadiazolyl)thiomethyl]-3-cephem-4-carboxylic acid was taken in 120 ml dichloromethane and to this 9.39 g of hexamethyl disilazane was added and refluxed for 2 hours to get a clear solution. After 2 hours the clear solution was cooled to 10°C and 12.6 g dimethylaniline was added to it and the reaction mixture was further cooled to -40°C.
C) Acylation:
   The reaction mixture A was cooled to -40°C, to which reaction mixture B was added and the temperature of the condensed mass was maintained at -25°C for 10-15 minutes. The quantification of condensed mass showed 53% of cefazolin.
   PMR(DMSO-d₆) δ ppm : 2.7(s,3H), 3.7(dd,2H) 4.35(dd, 2H), 5.15(d,1H), 5.4(s,2H), 5.8(q,1H), 9.39(s,1H), 9.6(d,2H).
D) Isolation of 7-(1-(1H)-tetrazolylacetamido)-3-[2- (5-methyl-1,3,4-thiadiazolyl)thiomethyl]-3-cephem-4-carboxylic acid.
   To the above condensed mass, 100 ml of water was added and the pH was brought to 6 by using triethylamine. The layer was separated and the pH of the aqueous layer was adjusted to 1.5 to yield white crystals of cefazolin. This was filtered and washed with water followed by methanol.
   a) IR : (Main bands) in cm ⁻¹
      3280 (-NH), 3140, 3075 (N=N, -C=N- tetrazole ring), 2620, 2580 (-OH, bonded, -COOH), 1770 (C=O lactam), 1715 (C=O acid), 1670 (C=O amide-I), 1555 (C=O amide-II).
   b) UV Characteristics :
      UV max = 272.5 nm (in water), E(1%, 1cm) = 290.76
   c) Specific rotation :
      [α]²⁰_{D} = -19.6° (C=1% aqueous solution)

### Example 10 :

### Preparation of 7-(1-(1H)-tetrazolylacetamido)-3-[2-(5-methyl-1,3,4-thiadiazolyl)thiomethyl]-3-cephem-4-carboxylic acid

A) Activation of 1H-tetrazol-1-acetic acid.
   Same procedure as in example 8 was followed. But, 140 ml of dichloromethane was used for activation.
B) Preparation of 7-amino-3-[2-(5-methyl-1,3,4-thiadiazolyl)thiomethyl]-3-cephem-4-carboxylic acid solution:
   20.0 g of 7-amino-3-(2-methyl-1,3,4-thiadiazol-5-yl) thiomethyl-3-cephem-4-carboxyllc acid was taken in 140 ml dichloromethane and hexamethyldisilazane was added and refluxed for two hours to get a clear solution. This solution was cooled to 10°C and 12.6 g of dimethylaniline was added. The resulting mixture was cooled to -40°C.
C) Acylation :
   The reaction mixture A was cooled to -40°C to which reaction mixture B was added and the temperature of the resulting condensed mass was maintained at -25°C for 10-15 minutes. The quantification of the condensed mass showed 61% of cefazolin.
D) Isolation of 7-(1-(1H)-tetrazolylacetamido)-3-[2- (5-methyl-1,3,4-thiadiazolyl)thiomethyl]-3-cephem-4-carboxylic acid.
   Same procedure as in example 9 was followed.

### Example 11 :

### Preparation of 7-(((2-aminothiazol-4-yl)(methoxyimino)acetyl)amino)-8-oxo-3-(((1,2,5,6-tetrahydro-2-methyl-5,6-dioxo-1,2,4-triazin-3-yl)thio)methyl) -5-thia-1-azabicyclo (4.2.0) oct-2-ene-carboxylic acid (ceftriaxone) :

A) Activation of 2-(2-aminothiazol-4-yl)-2-syn-methoxyimino acetic acid) :
   9.06 g of sulfuryl chloride was slowly added to 4.91 g of dimethylformamide at -25°c. The temperature was slowly raised to 0°c in 60-90 minutes. At this temperature white solid i.e. dimethyl forminium chloride chlorosulfate (DFCCS) crystallises out, 50 ml of dichloromethane was added to the solid crystals and stirred vigorously for 15-20 minutes when the solid crystals melt to form an immiscible white crystalline coloured oil. The immiscible oil was decanted in the lower protion and was slowly added to 11.37 g of 2-(2-aminothiazol-4-yl) methoxyimino acetic acid in 120 ml of dichloromethane cooled to -30°c in 20-30 minutes. The temperature was slowly raised to -10°c in one hour to get a clear solution.
B) 7-amino-3-desacetoxy-3-((2,5-dihydro-6-hydroxy-3-methyl-5-oxo-as-triazin-3-yl) thio) cephalosporanic acid solution :
   20.0 g of 7-amino-3-desacetoxy-3-((2,5-dihydo-6-hydroxy-3-methyl-5-oxo-as-triazin-3-yl) thio) cephalosporanic acid in 160 ml of dichloromethane. To the resulting reaction mixture 40 ml of N,O-bis-silyl acetamide was added and stirred for 8 hours at 30°c to get a clear solution, cooled to -20°c and 8.35 ml dimethylaniline was added to it and the reaction mixture was finally cooled to -50°c.
C) Acylation : The reaction mixture (A) was cooled to -30°c and added to the reaction mixture (B). The temperature was maintained at -40°c for 20-30 minutes.
   Thick precipitation occured which became thin during stirring, HPLC showed the formation of 70% of ceftriaxone.
   PMR (DMSO-d₆) δ ppm : 3.3(d), 3.59(s), 3.9(s), 4.2(d), 5.1(d), 5.72(d), 6.8(s), 9.7(d).
D) Isolation : After 30 minutes of the reaction, 200 ml of water was added at -40°c and stirred for 30 minutes at 20-25°c. The pH of the reaction mass was brought to 6.5 by adding triethylamine. The queous layer was separated and as treated with 5.0 g of activated carbon and 2.0 g of sodium bisulfate for 30 minutes, filtered and washed with 40 ml of water. The pH is then adjusted to 3.2 by 1:1 HCL, seeded and the resulting mixture was stirred for 30 minutes and then further stirred for two hours at 0°c to -5°c. The product was filtered, washed with chilled isopropyl alcohol to give 21g (70%) of Ceftriaxone, Assay = 92.88% - 95%.

### Example: 12

### Preparation of 7-[[(2-aminothiazol-4-yl)-2-syn-methoxyimino] acetamido]-8-oxo-3-[(1,2,5,6-tetrahydro-2-methyl-5,6- dioxo-1,2,4-triazin-3-yl)thiomethyl] cephalosporanic acid (ceftriaxone)

A) Activation of 2-(2-aminothiazol-4-yl)-2-syn-methoxyimino acetic acid.
   Same procedure as in example 11 was followed.
B) Quaternary salt of 7-amino-3-desacetoxy-3-(2,5-dihydro-6-hydroxy-3-methyl-5-oxo-as-triazin-3-yl)thio) cephalosporin acid.
   20.0 g of 7-amino-3-desacetoxy-3-(2,5-dihydro-6-hydroxy-3-methyl-5-oxo-as-triazin-3-yl)thio)cephalosporanic acid in 120 ml of dichloromethane was cooled to -15°C and 12.3 g of tetramethyl guanidine (TMG) was added to it. The temperature was slowly raised to -20°C to -25°C in one hour. The reaction mixture was stirred for one hour at 25°C to get a clear solution. This solution was further cooled to -30°C to -35°C and 8.35 ml dimethylaniline was added to it and the reaction mixture was finally cooled to -40°C.
C) Acylation :
   Same procedure as in example 11 was followed. HPLC showed formation of 75% of ceftriaxone:
D) Isolation.
   Same procedure as in example 11 was followed.

### Example 13 :

### Preparation of 7-[(2-aminothiazol-4-yl)(methoxyimino) acetamido]-8-oxo-3-[[(1,2,5,6-tetrahydro-2-methyl-5,6-dioxo-1,2,4-triazin-3-yl)thio]methyl] cephalosporanic acid (ceftriaxone)

A) Activation of 2-(2-aminothiazol-4-yl)-2-syn-methoxyimino acetic acid.
   Same as procedure as in example 11 was followed.
B) 7-amino-3-desacetoxy-3-[(2,5-dihydro-6-hydroxy-3-methyl-5-oxo-as-triazin-3-yl)thio]cephalosporanic acid solution.
   20.0 g of 7-amino-3-desacetoxy-3-(2,5-dihydro-6-hydroxy-2-methyl-5-oxo-as-triazin-3-yl)thio)cephalosporanic acid in 200 ml of dichloromethane was taken and 19.05 g of trimethylsilyl chloride(TMCS) was added to it, followed by refluxing for 3 hours to give clear solution, cooled to -20°C and 8.53 ml dimethylamine was added to it. The reaction mixture was finally cooled to -50°C.
C) Acylation:
   Same procedure as in example 11 was followed HPLC showed conversion to 70% of ceftriaxone.

### Example 14

### Preparation of 7-(((2-aminothiazol-4-yl) (methoxyimino)acetyl)amino)-8-oxo-3-(((1,2,5,6-tetrahydro-2-methyl-5,6-dioxo-1,2,4-triazin-3-yl)thiomethyl)-5-thia-1-azabicyclo (4.2.0) oct-2-ene-2-carboxylic acid (Ceftriaxone).

A) Activation of 2-(2-aminothiazol-4-yl)-2-syn-methoxyimino acetic acid :
   8.72 g. of sulfuryl chloride was slowly added to 4.7 g. of dimethyl formamide at -25°c. The temperature was raised slowly to 0°c in 60-90 minutes. Dimethyl forminium chloride chlorosulphate (DFCCS) crystallisis out at 0°c. 50 ml of dichloromethane was added to this solid crystals, which melts to get a colourless insoluble oil. This oil was decanted and taken separately. 11,37 g of 2-(2-aminothiazol-4-yl) methoxy imino acetic acid was taken in 120 ml dichlomethane and cooled to -30°c, to which the above said oil is added. The temperature was slowly raised to -10°c to get a clear solution and was kept at this temperature for 30 minutes.
B) Preparation of 7-amino-3-desacetoxy-3-((2,5-dihydro-6-hydroxy-3-methyl-5-oxo-as-triazin-3-yl) thio) cephalosporanic acid solution :
   20.0 g of dry 7-amino-3-desacetoxy-3-(2,5-dihydro-6-hydroxy-3-methyl-5-oxo-as-triazin-3-yl thio) cephalosporanic acid was taken in 120 ml of dichloromethane to which 13.05 g of Hexamethyldisilazane, 2.5 g of Trimethyl chloro silane and 0.5 g of imidazole were added and refluxed for 5-6 hours to get a hazy reaction mixture. This was cooled to 10°c and 8.3 ml of N,N-dimethyl aniline was added and was further cooled to -50°c.
C) Acylation :
   Reaction mixture (A) was cooled to -50°c and reaction mixture (B) was added to (A) to get a clear solution and the resulting temperature of the reaction mixture was maintained -45 to -50°c for 30 minutes. HPLC monitoring showed the formation of Ceftriaxone 86%.
D) Isolation :
   After 30 minutes, 200 ml of water and 50 ml of triethylamine was added to get pH 6.5 - 7.0. The layer was seperated and the aqueous layer was taken and 20 ml of Isopropyl alcohol and 40 ml of ethyl acetate was added and stirred for 10 minutes at 20-25°c. pH of this aqueous layer was adjusted to 2.5 by using Formic acid to get white crystalls. This was further cooled to 0-5°c and maintained for 1 hour. After 1 hour the product was filtered and washed with 50 ml of chilled water. The product was dried under vacuum to get white crystals of purity 98%.

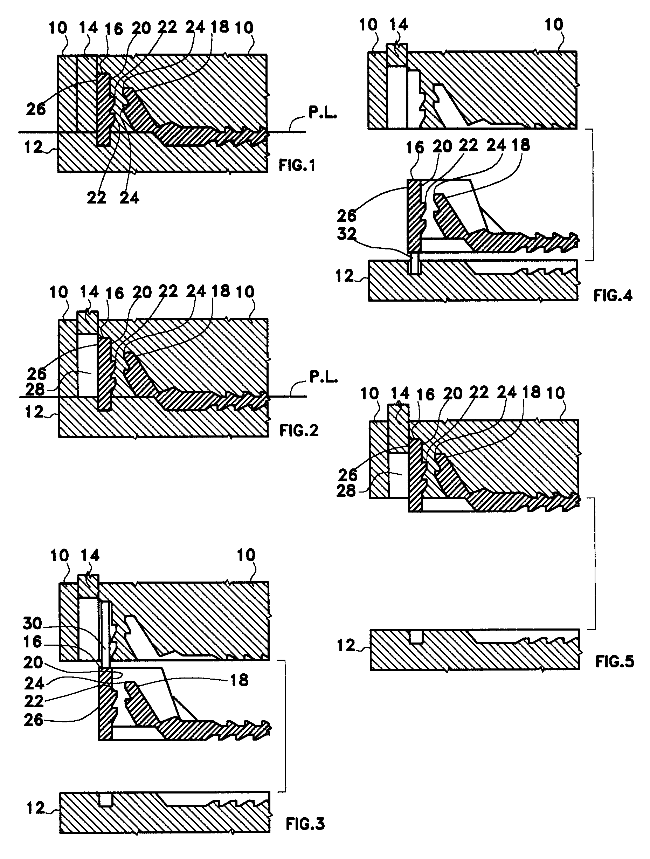

## Claims

1. The compound of formula I 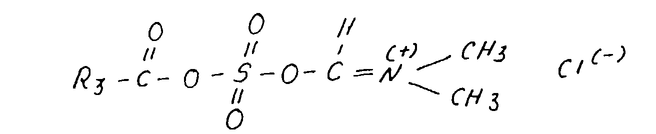
wherein, R₃ =

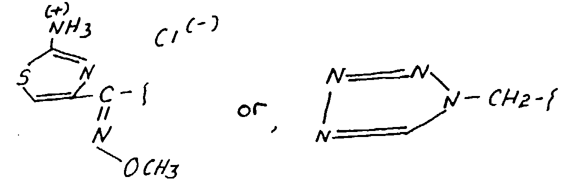

2. A process for the production of compound of formula I comprising -
reacting dimethyl formiminium chloride chloro sulphate (DFCCS) of formula XI, 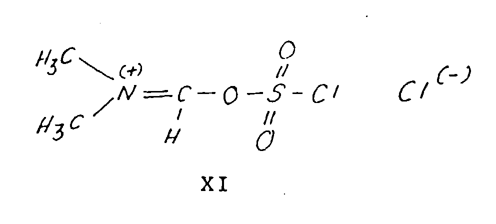 with 2-(2-aminothiazol-4-yl)-2-methoxyimino acetic acid or 1H-tetrazole-1-acetic acid in a solvent such as herein described at a temperature ranging from -30°C to -15°C, preferably at about -20°C.

3. A process as claimed in claim 2 wherein said solvent used is selected from dichloromethane, chloroform, benzene, toluene.

4. A process as claimed in any one of claims 2 or 3 wherein said DFCCS of formula XI is N,N-dimethyl formiminium chloride chlorosulphate obtained by reacting dimethyl formamide (DMF) with sulfuryl chloride, and
said compound of formula I is
a reactive derivative of formula Ia, 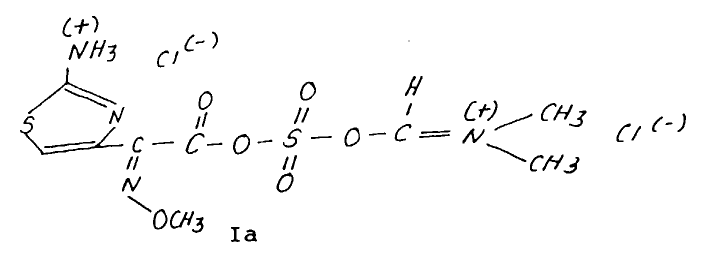 obtained by reacting said 2-(2-aminothiazol-4-yl)-2-methoxyimino acetic acid with said DFCCS of formula XI
or
is a reactive derivative of formula Ib, 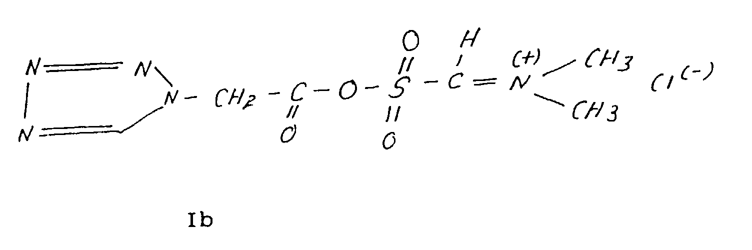 obtained by reacting said 1H-tetrazol-1-acetic acid with said DFCCS of formula XI.

5. A process as claimed in claim 4 wherein the molar ratio of said DFCCS with respect to said 2-(2-aminothiazol-4-yl)-2-methoxy imino acetic acid or said 1H-tetrazol-1-acetic acid is 1.1 to 1.2 preferably 1.17.

6. A process for the preparation of cephalosporin antibiotics of formula II 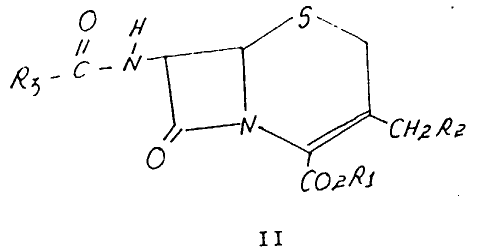
wherein R₁ = H, Carboxylic protecting group R₂ = acetoxy, 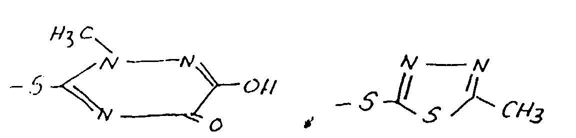 R₃ = 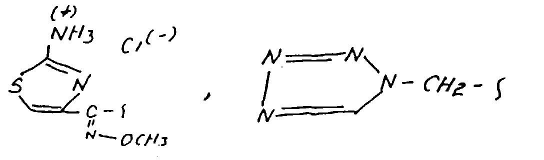
which comprises reacting silylated 7-amino cephalosporanic acid derivatives of formula X, 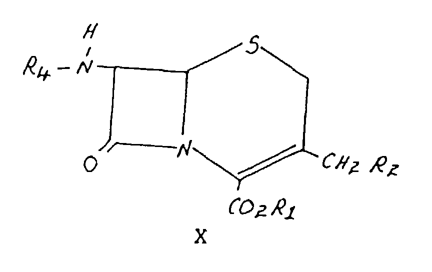 in which R₁, R₂ is as defined in said formula II; R₄ = silyl group or hydrogen; with a reactive derivative of formula I; in a solvent such as herein described at temperature ranging from -70°C to -30°C, preferably at about -55°C.

7. A process as claimed in claim 6, wherein the molar ratio of said reactive derivative of formula I with respect to said silylated 7-ACA is 1.1 to 2 preferably 1.2.

8. A process as claimed in any one of claims 6 or 7 wherein said reactive derivative of formula I is 2-(2-aminothiazol-4-yl)-2-methoxyimino acetyl sulfate dimethyl formiminium chloride according to formula Ia.

9. A process as claimed in claim 6 or claim 7 wherein said reactive derivative of formula I is 1H-tetrazol-1-acetyl sulphate dimethyl formiminium chloride according to formula Ib.

10. a process for the preparation of DFCCS of formula XI comprising reactive dimethyl formamide (DMF) with sulfuryl chloride.
